Europäisches Patentamt

European Patent Office

Office européen des brevets

(19)

(11) **EP 1 355 145 A1**

(12) **EUROPEAN PATENT APPLICATION**

(43) Date of publication:
**22.10.2003 Bulletin 2003/43**

(51) Int Cl.7: **G01N 21/67**, G01N 21/86,
G01N 33/20, G01J 3/443

(21) Application number: **03009038.5**

(22) Date of filing: **17.04.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK** | • **Zhijun, Yang**<br>**Beijing 100081 (CN)**<br>• **Xinsheng, Yang**<br>**Beijing 100081 (CN)**<br>• **Hongbin, Gao**<br>**Beijing 100081 (CN)**<br>• **Yunhai, Jia**<br>**Beijing 100081 (CN)** |
| (30) Priority: **19.04.2002 CN 02117115**<br>**02.12.2002 CN 02153706** | • **Liangjing, Yuan**<br>**Beijing 100081 (CN)**<br>• **Wenjun, Qu**<br>**Beijing 100081 (CN)** |
| (71) Applicant: **Central Iron & Steel Research Institute**<br>**Beijing 100081 (CN)** | • **Zhening, Weng**<br>**Beijing 100081 (CN)** |
| (72) Inventors:<br>• **Haizhou, Wang**<br>**Beijing 100081 (CN)**<br>• **Chen, Jiwen**<br>**Beijing 100081 (CN)** | (74) Representative: **DTS Zürich**<br>**Postfach 873**<br>**8029 Zürich (CH)** |

(54) **A method for analysing metals in the fundamental state utilizing the statistical distribution of elements**

(57) The present invention relates to a new analysis method to determine chemical compositions and states of metal materials. It includes the continuous steps of: using metallic discharge electrodes to perform the continuous spark discharge on the sample which is moved relative to the electrodes; dispersing the excited spark spectrum to form the linear spectrum with preset wavelength; recording the positions of every single spark discharge and the spectrum signals rapidly and at realtime; transferring the spectrum signals into electrical signals which are then inputted into signal memory; using a computer to statistically analyze the linear spectrum obtained from the single spark discharge, by which the distribution of chemical compositions, segregation, porosity and inclusion in the sample are resulted and then outputting these results. According to the present invention, the sample is subject to continuous excitation and synchronous scanning without pre-sparking, and every single spark spectrum signals is rapid collected and digital analyzed. Thus the original state of inclusion in sample without re-melting is obtained and various parameters for elements in sample, such as chemical compositions, segregation, porosity and inclusion content and distribution of these parameters are able to be simultaneously determined with one time scanning.

Fig.1

EP 1 355 145 A1

## Description

### Technical Field

The invention relates to the field of material analysis by means of testing physical properties of materials, in particular relates to the determination of chemical compositions and states of metal materials.

### Technical Background

Chemical composition, segregation, surface defection and the content of inclusion are four important indexes in quality control of iron and steel production. In the prior art, it is common to uses sulfur imprint experiment to test the segregation or distribution of elements in steel, acid leaching to test the central porosity and metallographic examination to test the inclusion shape and distribution. The above traditional methods are tedious and slow, and as a result difficult to be used to do quantitative analysis. (Sun Yeyin. *Optical microscopical analysis.* Beijing : Qinghua University Press, 1996, Oct.: p21-40).

In the prior art spark source atomic emission spectrometer is used to determine chemical compositions in materials, but only average content can be obtained, incapable of determining the composition distribution, not to mention the analysis of segregation, porosity, and quantitative distribution of inclusion. The main reason is that the spectrometer has the following three shortages among which the first is static excitation, which means that the sample is fixed during the analysis, only a spot of 5mm diameter is obtained in one excitation and the position of a single discharge can't be recorded, so that the distribution of elements and inclusion couldn't be analyzed. The second shortage of the spectrometer is the integration test mode. That means that the collected object is the voltage integrated according to several thousands of discharge pulses, so that a single spark character cannot be distinguished, and the chemical information, which hides in a single spark discharge, could not be resolved. The third shortage is high energy pre-spark. Because most of inclusions are re-melted after 20 to 30 seconds pre-spark, therefore abnormal sparks from which the information of inclusion can be obtained could not be observed.

### Brief Description of the Invention

The purpose of this invention is to provide a method for analyzing statistic distribution of some parameters of a metal material, which, without pre-spark, can directly determine the distribution of chemical composition, composition distribution of elements, segregation, porosity and inclusion in metal materials simultaneously.

The above-mentioned purpose is solved by the following solution:

According to the present invention, a method for spectral analysis of the statistic distribution of some parameters of a metal in its original position comprises the following procedures:

Excitation step, i.e. to excite samples continuously with exciting source and to locate each spark discharge by synchronously scanning;

Light dispersion step, i.e. to disperse the excited spectrum to form a linear spectrum;

Signal collection step, i.e. to collect and record the position signal and spectral intensity of a single spark discharge rapidly; and

Signal analysis step, i.e. then the results are outputted to perform statistic resolution and quantitative analysis according to the collected and stored linear spectral intensity, therefore the distribution of the chemical composition, segregation, porosity and inclusion in samples are determined, an then the results are outputted.

The present the invention will be described below in detail.

1. Excitation step, in which exciting source is used to excite samples continuously and each spark discharge is located by synchronously scanning.

The sample is held on a system for continuous excitation and synchronous scanning (a kind of programmable automatic scanning table equipment) during the spark excitation discharge, the position of a single spark discharge is recorded in real-time by a detector simultaneously, and a relative zero point for localization is given. Sample and electrode are moved relative to each other along X or Y axes on a two-dimensional plane or a circle at the speed of 0.1~1mm/sec. Samples are continuously excited by spark discharge dynamically during the movement without pre-sparking, so that no inclusion is re-melt, and spark spectrum of sample in its original position could be obtained.

2. Light dispersion step, in which the excited spectrum is dispersed to form the linear spectrum

The spectrum, produced from sample excitation by excitation source system, enters optical dispersion system through entrance slits of monochrometer and then is dispersed to form a linear spectrum with the wavelengths range of 120~800nm. Then they enter the collection system of spectral intensity for a single spark discharge through exit slits with various wavelength ranges. The number of exit slits is in the range of 3~55, i.e. three to fifty five linear spectrum channels are adopted, and many elements in the sample can be analyzed simultaneously.

3. Signal collection step, in which the spectral intensity of a single spark discharge is collected and recorded

The linear spectrum coming out of the exit slits arrives at the photoelectric multiplier and is converted into electric signals. After being amplified, the signals are A/D converted by the collection board and then, recorded and stored in digital form. The collection speed can reach 1~200kHz/channel. Integration procedure for thousands of spark electric signals in the prior art is eliminated, and the collection and record of the single spark discharge's spectrum intensity can be realized.

4. Signal analysis step, in which statistic resolution and quantitative analysis for the collected and stored linear spectral intensity are performed.

The collected and stored spark discharge linear spectrum intensity is transferred into computer, it is statistically resolved by the executable program which has been stored in the computer memory, so the distribution of chemical composition, segregation, porosity and inclusion of the sample could be determined.

According to the present invention, the continuous moving excitation mode without pre-sparking for the excitation light source is used. The quantitative formula for statistic resolution and quantitative analysis corresponding to a single discharge is as follows:

$$R_i = I_{a,i} / I_{r,i} = KC_i^b$$

where,

$R_i$ is spectral intensity ratio measured in number i discharge measurement, $I_{a,i}$ and $I_{r,i}$ are intensities of respectively analytical lines and reference lines measured in number i measurement respectively, $C_i$ is element content at the point of number i excitation (i.e. the content obtained by measurement), b is a constant relative to spectral line character which the value is in the range of 0~1, and K is a parameter relative to sample evaporation, excitation process and sample composition etc.

More than three reference samples are excited by non-pre-spark continuous moving excitation mode before unknown sample is analyzed. The intensity ratio of analytical and reference lines is recorded. K and b values are calculated by means of the above quantitative formula. Then under the same conditions, unknown samples are excited and the intensity ratio of analytical and reference lines is recorded. From the above quantitative formula, the element content of number i point in the sample is obtained.

The present invention is mainly beyond the analysis method of prior art such as: 1) the collected spectral intensity of the present method is that of one single discharge, instead of the sum of total intensity from thousands discharge pulses that is the way used in the prior art; 2) the internal standardization of the present method is the intensity ratio of analytical and reference lines at the same measurement time, but the integral intensity ratio of analytical and reference lines during a period of time as in the prior art; 3) the element content obtained by the present method is that of one single discharge excitation spot (micron grade), but the excitation spot content which is re-melted by thousands of discharge pulses (millimeter grade) as in the prior art.

The present invention will be detailed described in conjunction with embodiments in which different parameters are measured respectively.

4.1 Chemical composition

According to the present invention, the chemical composition is calculated by use of the statistic average of element content corresponding to every single discharge in the scanned range. The quantitative calculation formula is shown as:

$$\overline{C} = \frac{\sum\limits_{1}^{n} C_i}{n}$$

where,
$\overline{C}$ is statistic average of element content in sample; and Ci is element content of number i spot in sample.

4.2 Element segregation

The element segregation of sample can be estimated by ratio of the highest content to average content over every spot for an element. The quantitative formula is as follows:

$$S = C_{max} / C_0 \ (or \ (C_{max} - C_0)/C_0)$$

The segregation is described by the frequency distribution of a single discharge for different content in the whole scanned area. The detailed operation mode refers to Example 2.
Where,
S represents the segregation of the element, $C_{max}$ is the highest content on the scanned line or surface; and $C_0$ is the average content over the scanned line or surface.

4.3 Porosity

Porosity of material is described by the intensity distribution, or the apparent density converted from the intensity distribution, of the matrix element (such as iron), that is, the spark discharge intensity distribution of sample is contrasted with that of pure iron. The obtained value is the sample porosity.

4.4 Inclusion content

Inclusion content in sample is resulted from calculating the proportion of the abnormal sparks to total sparks. The quantitative formula is as follows:

$$C_{insol} = C \cdot n/N$$

where,
$C_{insol}$ is inclusion content of non-metal of an element, C is content of the element in the sample, n is the number of single discharge for non-solid-solution, and N is the number of total single discharge.
The abnormal spark signal of inclusion is determined by the "threshold value", which is 1.5~2.0 times of the central value of the Gauss Distribution of blank sample intensity vs. frequency. The "threshold value" can be adjusted according to different instruments and samples. The detected signal which value is higher than "threshold value" is abnormal spark inclusion.
Type of inclusion can be determined by multi-channel synchronous resolution method. When discharge is occurring on inclusion, the spectral line intensity of inclusion element is enhanced enormously because of the local concentration of inclusion. A combination resolution can be carried out by preset composition of inclusion types. For example, after measuring abnormal signals of single discharge produced by all composition elements (oxygen, aluminum, nitrogen, et al.) in different type of aluminum inclusion, classification, qualitative and quantitative analysis for aluminum inclusion can be carried out by combination resolution of the spark behavior differences at different state of each element.
The size distribution of inclusion is described by the frequency distribution of the abnormal single discharge intensity from each channel. The frequency distribution of abnormal spark intensity from different inclusion size is corresponding to the size distribution of inclusion.
Abnormal signal of inclusion spark spectral intensity is related to the size of inclusion. The abnormal degree is increased by the size of inclusion. Average size of non-metal inclusion can be determined by subtraction of average intensity over non-solid-solution from that over inclusion signals. Inclusion size distribution in each sample is determined by the proportion of numbers of inclusion signals appeared in various spectrum intensities section to the total numbers of inclusion signals

The analytical results can be outputted in the following mode:

Distribution of element composition is represented by a 2D or 3D diagram, by means of the intensity (or element content) and the position of single spark discharge for each element as parameters.

Inclusion distribution is represented by a 2D or 3D diagram, by means of the single discharge intensity for non-solid-solution metal or the content and position for inclusion as the parameters.

Compared with the closest prior art, in the method according to the present invention, the sample is continuous excited without pre-sparking and synchronous scanned, and then the single spark spectrum signals are rapidly collected and performed digital resolution analysis, the original performance of inclusion in sample without re-melting can be obtained. In addition, the method according to the present invention has the following advantages:

1. Quantitative analytical results of all element contents, element composition distribution, segregation, porosity and inclusion in material can be obtained by one time of scanning analysis simultaneously. The testing information is complete and highly contrastable.

2. Chemical composition of sample is calculated by use of element average content over all points in scanned area, so that the error caused by the difference of sampling point due to element segregation is avoided.

3. Composition contents of each element at random point or line of the material can be displayed by 2D diagram and the most segregative position is marked. The quantitative result is accurate. The practicability of the proposed method is good.

4. Porosity state at random point or line of material can be displayed by 2D diagram and the most porose position is marked. The quantitative result is accurate. The practicability of the proposed method is good.

5. The distribution of element and inclusion in material can be displayed by three-dimensional diagram, which shows an intuitionistic image.

6. The proposed method only needs a short analytical periods and suitable for monitoring and control on the production site.

**Brief Description of the Drawing**

FIG 1 is a 3-Dimentional diagram showing carbon content in sample A2 in an example of this invention. Where, X-axis and Y-axis express displacement of sample during moving scanning (mm); Z-axis expresses spectral line intensity, i.e. element content.

FIG 2 is 2-Dimensional contour plan showing carbon content in sample A2 in the example.

FIG 3 is 2-Dimensional contour plan showing carbon content in sample A1 in the example.

FIG 4 is a distribution plan of manganese content in the melting-dressing area between welding lines in sample C in the example.

FIG 5 is statistic distribution plan of carbon's segregation in sample D.

FIG 6 is statistic distribution plan of carbon's segregation in sample E.

FIG 7 is size distribution plan of $Al_2O_3$ inclusion in sample F.

**Example 1 Distribution of chemical composition, segregation, porosity and inclusion in sample**

The chemical composition, segregation, porosity and distribution of inclusion in three samples were analyzed by said method for analyzing metals in original position by means of statistic distribution according to the present invention.

Three samples were as follows:

Sample A was carbon steel slab from continuous-casting, in which A1 had been pressed lightly by roller after the slab was conveyed out of the continuous casting machine and A2 had not been pressed lightly; sample B was 20MnSi steel billet; sample C was low-carbon steel welding-line metal used for ship.

Chemical compositions of sample A, B and C designed in metallurgy are listed in Table 1.

A piece was cut out from the above sample as test sample which was worked to get a plane, on which then a fresh face was grinded using sand paper or by grinder. The test sample was held on the localization system for continuous excitation and synchronous scanning (An analyzer for analyzing a metal in its original position, reference to Patent Application of the People's Republic of China, Appl. No.: 02116294.8, filing date April 1, 2002)

Before spark spectrum exciting, the exciting optical source system was cleaned by argon, the test sample was held on the localization system for continuous excitation and synchronous scanning, and a relative zero point was determined.

Then the exciting optical source system was started to implement continuous excitation without pre-sparking and moving-scanning. The test sample scanning was line-type plane scanning, along X-axis it was carried out continuously at the speed of 1mm/sec; along Y-axis was carried out step-by-step.

The exciting parameters of spark spectrum for the three test samples were respective as follows: frequency, 500Hz; inductance, 130 $\mu$ H; capacitance, 2.2 $\mu$ F; resistance, 1.0 $\Omega$; gap, 2.0mm; electrode material, 45° top angle line tungsten electrode with a 5mm diameter.

The spectrum produced by continuous moving exciting enters optical dispersion system through entrance slits of monochrometer and then was dispersed by the grating to form a linear spectrum. The spectrum was further dispersed by the monochrometer, and then was, at last, collected by detect system though eight exit slits. The collecting speed of linear spectrum signals for single spark discharge was 100KHz per channel. The spectrum signals was recorded as digital mode after A/D transformation and stored in the computer. The determinations of element contents were completed after dealing with by the executable program.

The wavelengths and elements' contents for the three samples are listed in table 2. The reference element for determination is iron.

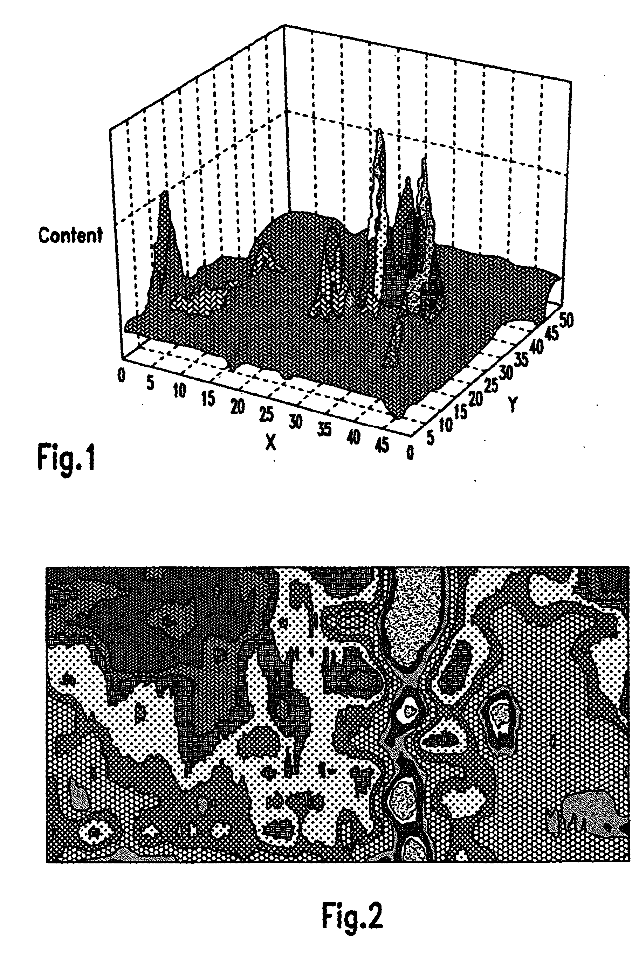

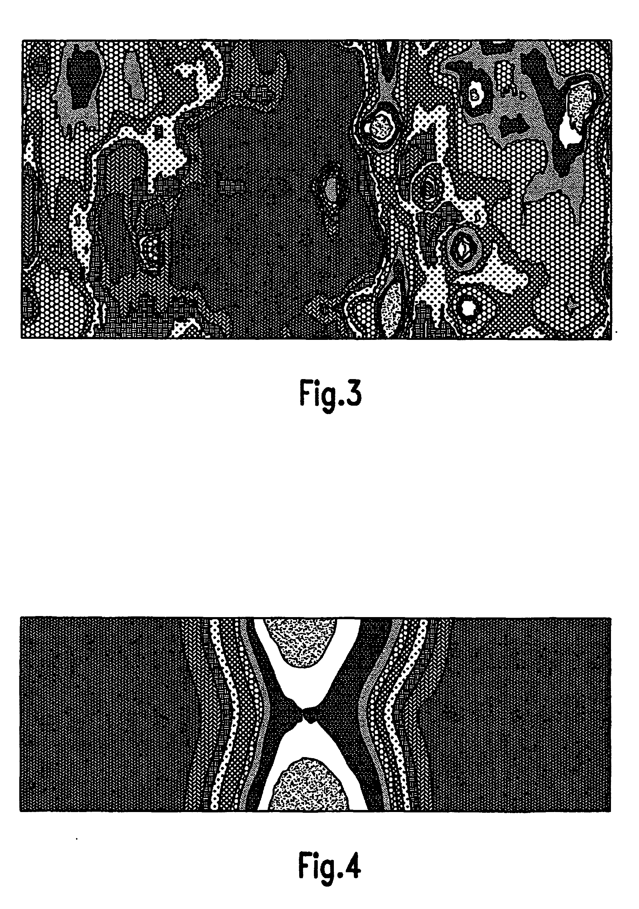

After determination of elements' content, carbon content in sample A2 was displayed in a 3-dimentional diagram (Fig. 1) and carbon content in sample A1 and A2 was displayed in a 2-dimentional contour (Fig. 4 and 3).

Fig.1 is showing the distribution of carbon element and segregation of chemical composition in sample.

### Table 1   Chemical composition of samples preset in metallurgy in the example

Weight %

| element No. of sample . | C | Si | Mn | P | S | Al | Cu | Fe |
|---|---|---|---|---|---|---|---|---|
| A₁.A₂ | 0.2 | 0.07 | 0.6 | 0.02 | 0.05 | 0.06 | 0.05 | 99 |
| B | 0.2 | 0.3 | 1.1 | 0.02 | 0.02 | 0.002 | 0.2 | 98 |
| C | 0.07 | 0.3 | 0.4 | 0.01 | 0.003 | 0.05 | 0.006 | 99 |

Table 2　Element contents (wt%) and wavelengths for the three samples in the example

| No. of Sample | Element | C | Si | Mn | P | S | Al | Cu | Fe |
|---|---|---|---|---|---|---|---|---|---|
| A1 | Wavelength, nm | 193.0 | 288.1 | 293.3 | 178.3 | 180.7 | 394.4 | 327.3 | 187.7 |
| | Content, % | 0.195 | 0.077 | 0.63 | 0.026 | 0.035 | 0.055 | 0.057 | residure |
| | Segregation | 1.18 | 1.17 | 1.10 | 1.19 | 1.17 | 2.00 | 1.32 | / |
| | Inclusion | /- | / | / | / | / | $Al_2O_2$+AlN 0.0035 | / | / |
| | Porosity | / | / | / | / | / | / | / | 7.40 |
| A2 | Wavelength, nm | 193.0 | 288.1 | 293.3 | 178.3 | 180.7 | 394.4 | 327.3 | 187.7 |
| | Content, % | 0.205 | 0.075 | 0.65 | 0.025 | 0.050 | 0.061 | 0.055 | residure |
| | Segregation | 1.51 | 1.33 | 1.17 | 1.80 | 1.30 | 1.97 | 1.82 | / |
| | Inclusion | / | / | / | / | / | $Al_2O_3$+AlN 0.0035 | / | / |
| | Porosity | / | / | / | / | / | / | / | 7.30 |
| B | Wavelength, nm | 193.0 | 288.1 | 293.3 | 178.3 | 180.7 | 394.4 | 327.3 | 187.7 |
| | Content, % | 0.23 | 0.33 | 1.13 | 0.017 | 0.027 | 0.0022 | 0.21 | residure |
| | Segregation | 2.16 | 1.49 | 1.67 | 1.25 | 2.24 | 1.32 | 1.27 | / |
| | Inclusion | / | / | MnS 0.023 | / | / | / | / | / |
| | Porosity | / | / | / | / | / | / | / | 7.21 |
| C | Wavelength, nm | 193.0 | 288.1 | 293.3 | 178.3 | 180.7 | 394.4 | 327.3 | 187.7 |
| | Content, % | 0.073 | 0.292 | 0.441 | 0.011 | 0.0032 | 0.047 | 0.0056 | residure |

**Example 2 Analysis of composition segregation in sample**

The frequency distribution of carbon segregation in two samples are analyzed by said analytical method according to the present invention.
Both sample D (sample No. 24241) and sample E (sample No. 26796) were carbon steel slab from continuous-casting, in which the average carbon content was 0.223% in sample D and 0.227% in sample E.

A test sample (140mm×50mm) cut out from the sample, was processed to get a plane, on which then a fresh face was grinded using sand paper or by grinder. The test sample was held on the localization system of synchronous scanning for continuous Excitation that is the same as Example 1.

Before spark spectrum exciting, the exciting optical source system was cleaned by argon. The test sample, which was held on the localization system of synchronous scanning for continuous Excitation, was excited continuously and a relative zero point was determined.

Then the exciting optical source system was started up to implement continuous Excitation without pre-sparking and moving scanning. The test sample scanning was line-type plane scanning and continuous scanning along X-axis. The scanning speed was 0.1mm/sec. The scanning along Y-axis was step-by-step.

The exciting parameters of spark spectrum for the two test samples are as follows:

Frequency, 500Hz; inductance, 130 μ H; capacitance, 2.2 μ F; resistance, 1.0 Ω; gap, 2.0mm; electrode material, 45° top angle line tungsten electrode with a 5mm diameter.

The spectrum, produced by continuous moving scanning, enters optical dispersion system through entrance slits of monochrometer and then was dispersed into line-type spectrum by the grating. The spectrum is further dispersed by the monochrometer, then was, at last, collected by detect system though exit slits (wavelength of aluminum was 193.1 nm, and reference element iron 273.1 nm). The collecting speed of linear spectrum signals for single spark discharge is 50KHz per channel. The spectrum signals was recorded as digital form after A/D transformation and stored in the computer. The determination of carbon contents at every spots in the test sample was completed after dealing with by the executable program.

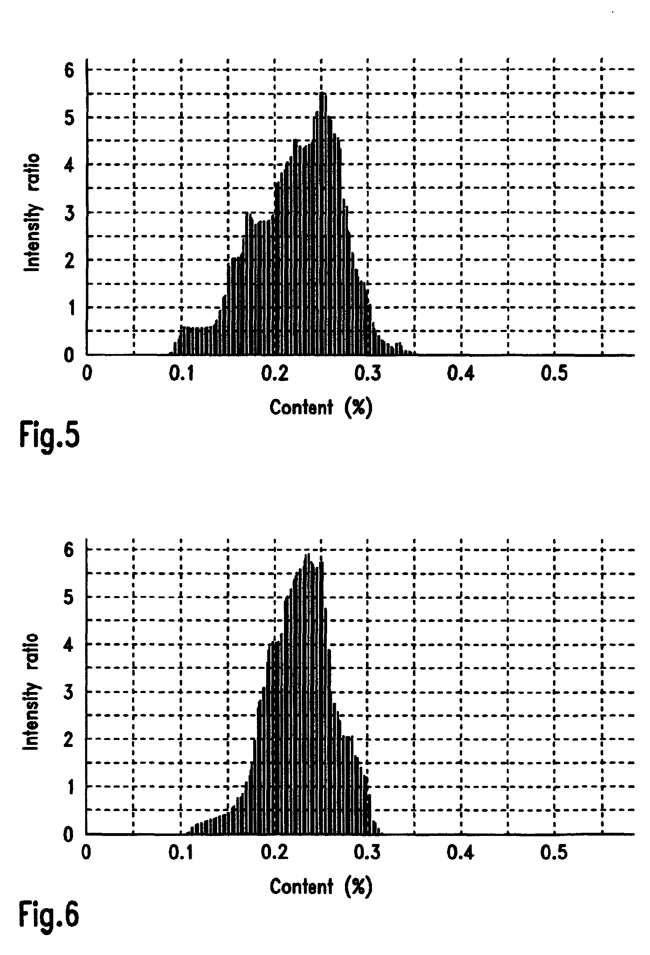

Fig. 5 and Fig. 6 are frequency distribution plan of carbon's segregation in sample D and sample E respectively.

It can be seen that carbon content within 28.98% area is only in range from 0.203% to 0.243% for sample D and carbon content within 42.97% area is in range from 0.207% to 0.247% for sample E. it is illuminated that distribution homogenization of carbon element in sample E is better than that in sample D.

## Example 3 Distribution of inclusion size in sample

The distribution of alumina inclusion size in sample was analyzed by said analytical method for original position statistic distribution of metals in this invention.

Sample F (sample No. 30#) was carbon steel, in which total aluminum content was 0.0362%.

A test sample (50mm×50mm) cut out from the sample, was processed to get a plane, on which then a fresh face was grinded using sand paper or by grinder. The test sample was held on the locating system of synchronous scanning for continuous Excitation the same as Example 1.

Before spark spectrum exciting, the exciting optical source system was cleaned by argon. The test sample, which was held on the localization system of synchronous scanning for continuous Excitation, was excited continuously and a relative zero point was determined.

Then the exciting optical source system was started up to implement continuous Excitation without pre-sparking and moving-scanning. The test sample scanning was linear plane scanning and continuous scanning along X-axis. The scanning speed was 0.3mm/sec. The scanning along Y-axis was step-by-step.

The exciting parameters of spark spectrum for the two test samples was as follows:

Frequency, 500Hz; inductance, 130 μ H; capacitance, 2.2 μ F; resistance, 1.0 Ω; gap, 2.0mm; electrode material, 45° top angle line tungsten electrode with a 5mm diameter.

The spectrum, produced by continuous moving scanning, enters optical dispersion system through entrance slits of monochrometer and then was dispersed into linear spectrum by the grating. The spectrum is further dispersed by the monochrometer, and then was, at last, collected by detect system though exit slits (wavelength of aluminum was 193.1nm, and reference element iron 273.1nm). The collecting speed of linear spectrum signals for single spark discharge is 50KHz per channel. The spectrum signals was recorded as digital form after A/D transformation and stored in the computer. The determination of aluminum contents at each spots in the test sample was completed after dealing with by the executable program.

a) Identification of noise signal and inclusion signal: The 1.6 times of central value in Guss Distribution (Normal Distribution) of intensities from a blank sample versus frequency was set up as "threshold value" and the value which was higher than that is distinguished inclusion signal.

b) The difference between the average intensity of inclusion signals and the "threshold value" was estimated as an average size of non-metal inclusion. The result was 11.61μ m.

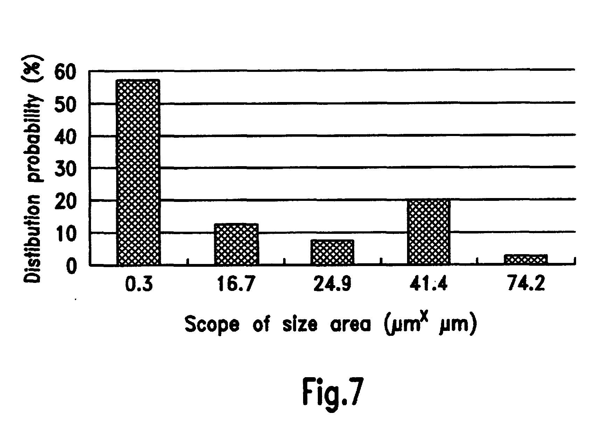

c) Inclusion size distribution in each sample was determined by the proportion of numbers of inclusion signals appeared in various spectrum intensities section to the total numbers of inclusion signals (Fig. 7).

**Claims**

1. A method for analysis the statistic distribution of parameters of the metal in its original position, **characterized in that** it includes the following steps which are continuously completed:

   Excitation, which excites samples continuously with exciting source and locates each spark discharge by synchronously scanning;

   Light dispersion, which disperses the excited spectrum to form a linear spectrum;

   Signal collection, which collects and records the position and the spectrum signal of a single spark discharge rapidly and at real-time, transfers the spectrum signal into electrical signal and then inputted into signal memory; and

   Signal analysis, which performs the statistic analysis of linear spectrum signal of said single spark, obtains the chemical composition, segregation, porosity and inclusion of said determined samples, and outputs the results.

2. A method of claim 1, **characterized in that** said sample and the excitation discharge electrode are moved relative to each other in two-dimensional X-Y direction or circumferential direction.

3. A method of claim 1, **characterized in that** said moving speed of the sample relative to the electrode is 0.1 to 1mm/sec.

4. A method of claim 1 or 2, **characterized in that** said excitation step is performed in a continuous and moving mode without pre-sparking.

5. A method of claim 1, **characterized in that** in said the light dispersing step, many spectrum channels of interest are formed by means of corresponding exit-slits so that a lot of elements can be determined simultaneously.

6. A method of claim 1 or 5, **characterized in that** said number of exit-slits is from three to fifty-five.

7. A method of claim 1, **characterized in that** said signal collecting step includes: conversion the optical signals from exit slits, i.e. the linear spectrum intensity of single spark discharge, into electrical signals, which are further transferred into digital signals after amplifying, then to record and store.

8. A method of claim 1 or 6, **characterized in that** the signal collecting speed of linear spectrum is in the range of 50 to 200kHz per channel.

9. A method of claim 1, **characterized in that** said signal analysis step comprises the step of: through a executable program in computer memory to perform the statistically resolution and quantitative analysis of linear spectrum intensity of said single spark discharge which are recorded and stored, in which the quantitative formula for corresponding single discharge is as follows:

$$R_i = I_{a,i} \,/\, I_{r,i} = KC_i^b$$

where,

   $R_i$ is spectral intensity ratio of number i measurement of single discharge;

   $I_{a,i}$ and $I_{r,i}$ are intensities of analytical and reference lines of number i measurement, respectively;

   $C_i$ is element content at the point of number i excitation (i.e. the content obtained by measurement);

   b is a constant relative to spectral line character, which value is between 0 and 1; and

   K is a parameter relative to sample evaporation, excitation process and sample composition etc.

**10.** A method of claim 1, **characterized in that** in the said signal analysis step, the chemical compositions of sample are resulted from calculating an average over the element contents from all spots within scanning scope, in which the quantitative formula is as follows:

$$\overline{C} = \frac{\sum_{1}^{n} C_i}{n}$$

where,

$\overline{C}$ is statistic average of element content in the sample; and

Ci is element content of number i spot in sample.

**11.** A method of claim 1, **characterized in that** in said signal analysis step, the segregation of sample is estimated by ratio of the highest content to the average content over all spots for an element, in which the quantitative formula is as follows:

$$S = C_{max}/C_0 \ (\text{or} \ (C_{max}-C_0)/C_0)$$

where,

S represents the segregation of the element;

$C_{amx}$ is the highest content on the scanning line or surface; and

$C_0$ is the average content over the scanning line or surface.

**12.** A method of claim 1, **characterized in that** in said signal analysis step, the sample porosity is defined by the intensity distribution of the matrix element which is then converted into an apparent density.

**13.** A method of claim 1, **characterized in that** in said signal analysis step, the inclusion content in sample is resulted from calculating the proportion of the abnormal sparks to total sparks, in which the quantitative formula is as follows:

$$C_{insol} = C \cdot n/N$$

where,

$C_{insol}$ represent the inclusion content of non-metal of an element;

C is content of the element in the sample;

n is number of single discharge for non-solid-solution; and

N is number of total single discharge.

**14.** A method of claim 1, **characterized in that** in said signal analysis step, the types of the inclusions are identified by multi-channel synchronous combined analysis and the size distribution of inclusion is described by the frequency distribution of the abnormal spark intensity at each channel.

**15.** A method of claim 1, **characterized in that** in the said signal analysis step, the outputted results are displayed through the distribution of element composition represented by a 2D or 3D diagram, by means of the intensity (or element content) and the position of single spark discharge for each element as parameters.

**16.** A method of claim 1, **characterized in that** in the said analysis step, the outputted results are displayed through the inclusion distribution represented by a 2D or 3D diagram, by means of the intensity and the position of single spark discharge for inclusion as the parameters.

Fig.1

Fig.2

Fig.3

Fig.4

Fig.5

Fig.6

Fig.7

**European Patent Office**

## EUROPEAN SEARCH REPORT

Application Number

EP 03 00 9038

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 504 933 A (SHIMADZU CORP) 23 September 1992 (1992-09-23) | 1,5-16 | G01N21/67 G01N21/86 G01N33/20 G01J3/443 |
| Y | * column 3, line 23 - column 6, line 15; figures 1,3 * | 2-4 | |
| Y | PATENT ABSTRACTS OF JAPAN vol. 007, no. 142 (P-205), 22 June 1983 (1983-06-22) & JP 58 055736 A (SHIMAZU SEISAKUSHO KK), 2 April 1983 (1983-04-02) * abstract * | 2,3 | |
| Y | EP 0 396 291 A (FISONS PLC) 7 November 1990 (1990-11-07) * column 1, line 45 - column 2, line 8 * * column 3, line 32 - line 46 * * column 6, line 52 - column 7, line 3 * * column 9, line 29 - line 51; figure 1 * | 4 | |
| X | PATENT ABSTRACTS OF JAPAN vol. 1999, no. 11, 30 September 1999 (1999-09-30) & JP 11 160240 A (KAWATETSU TECHNO RES CORP), 18 June 1999 (1999-06-18) * abstract * | 1,5-7 | TECHNICAL FIELDS SEARCHED (Int.Cl.7) G01N G01J |
| Y | EP 0 125 171 A (SIDERURGIE FSE INST RECH) 14 November 1984 (1984-11-14) * page 1, line 11 - line 13 * * page 4, line 10 - page 6, line 35; figure 1 * | 4 | |
| A | DE 197 53 348 A (SPECTRO ANALYTICAL INSTR GMBH) 10 June 1999 (1999-06-10) * column 1 - column 2; figure 1 * | 1,9-15 | |

The present search report has been drawn up for all claims

| Place of search | Date of completion of the search | Examiner |
|---|---|---|
| MUNICH | 24 July 2003 | Duijs, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 03 00 9038

This annex lists the patent family members relating to the patent documents cited in the above-mentioned European search report.
The members are as contained in the European Patent Office EDP file on
The European Patent Office is in no way liable for these particulars which are merely given for the purpose of information.

24-07-2003

| Patent document cited in search report | | Publication date | Patent family member(s) | | Publication date |
|---|---|---|---|---|---|
| EP 0504933 | A | 23-09-1992 | JP | 2032647 C | 19-03-1996 |
| | | | JP | 4294258 A | 19-10-1992 |
| | | | JP | 7050033 B | 31-05-1995 |
| | | | CN | 1065337 A ,B | 14-10-1992 |
| | | | DE | 69204872 D1 | 26-10-1995 |
| | | | DE | 69204872 T2 | 15-05-1996 |
| | | | EP | 0504933 A2 | 23-09-1992 |
| | | | US | 5303025 A | 12-04-1994 |
| JP 58055736 | A | 02-04-1983 | JP | 1002214 B | 17-01-1989 |
| | | | JP | 1521410 C | 12-10-1989 |
| EP 0396291 | A | 07-11-1990 | AT | 114815 T | 15-12-1994 |
| | | | DE | 69014398 D1 | 12-01-1995 |
| | | | DE | 69014398 T2 | 20-04-1995 |
| | | | EP | 0396291 A2 | 07-11-1990 |
| | | | JP | 2297046 A | 07-12-1990 |
| | | | US | 5285251 A | 08-02-1994 |
| JP 11160240 | A | 18-06-1999 | NONE | | |
| EP 0125171 | A | 14-11-1984 | FR | 2545609 A1 | 09-11-1984 |
| | | | AT | 29303 T | 15-09-1987 |
| | | | DE | 3465783 D1 | 08-10-1987 |
| | | | EP | 0125171 A2 | 14-11-1984 |
| DE 19753348 | A | 10-06-1999 | DE | 19753348 A1 | 10-06-1999 |

EPO FORM P0459

For more details about this annex : see Official Journal of the European Patent Office, No. 12/82